# EUROPEAN PATENT APPLICATION

(11) **EP 3 901 244 A1**
(43) Date of publication of application: **27.10.2021**
(21) Application number: 21156606.2
(22) Date of filing: 11.02.2021
(51) Int. Cl.: C12M 3/00, C12M 1/00, C12M 1/36, B01L 1/00

(54) **CELL CULTURE SYSTEM**

(30) Priority: 27.02.2020 JP 2020031324
(71) Applicant: Shibuya Corporation, Kanazawa-shi, Ishikawa 920-8681 (JP)
(72) Inventor: KIKUCHI, Atsunori, KANAZAWA-SHI, Ishikawa 920-8681 (JP)
(74) Representative: Mewburn Ellis LLP

(57) **Abstract**

A cell culture system 1 including: self-propelled transport device 11 for transporting cells or materials to be used for culture operation; treatment units U for performing culture operation using the cells and materials; and transport command unit Ca for controlling the self-propelled transport device 11 to move the cells and materials to a required treatment unit U. To control the self-propelled transport device 11, the transport command unit Ca uses cell driving parameters when the cells are moved, and uses material driving parameters when the materials are moved. Hence, transport of cells and materials using the self-propelled transport device can be optimized, and culture operation can be performed efficiently.

## Description

### BACKGROUND OF THE INVENTION

### Field of the Invention

The present invention relates to a cell culture system, more particularly to a cell culture system in which cells and materials used for culture operation are transported between a plurality of treatment units by self-propelled transport device.

### Description of the Related Art

Nowadays, a regenerative medicine-based treatment method that cultures cells recovered from a patient and uses the cells for treatment is known and a cell culture system that performs culture operation for efficiently culturing cells has been accordingly proposed.

The cell culture operation is composed of various treatments, and if all the culture operation is performed inside one isolator, the culture operation for other cells cannot be performed until all the treatments are completed, which causes the problem that a large number of cells cannot be cultured.

For this reason, a culture system is known provided with a plurality of treatment units configured to perform part of the aforementioned culture operation so that cells can be sequentially transported between the treatment units according to the treatment order of the aforementioned culture operation (Japanese Patent Laid-Open No. 2020-194).

In Japanese Patent Laid-Open No. 2020-194, cells can be transported by a plurality of self-propelled transport device, and each self-propelled transport device is individually controlled by transport command unit, so that the culture operation is divided among the treatment units and the cells are automatically transported.

In Japanese Patent Laid-Open No. 2020-194, materials such as pipettes and centrifuge tubes used for culture operation and culture broth used for culturing are also transported with self-propelled transport device to be supplied to each treatment unit.

When the cells are transported using the self-propelled transport device, the cells may be stressed by vibration or impact, or the culture broth may spill out of the culture vessel. It is therefore necessary to control the movement of the cells so that they move gently during transportation using the self-propelled transport device.

Traditionally, materials have also been transported under the same control as cells, which causes a problem of time loss due to transportation during time-consuming treatment such as supplying a large amount of material to a treatment unit.

To solve such a problem, the present invention provides a cell culture system that can appropriately transport cells and materials.

### SUMMARY OF THE INVENTION

In particular, a cell culture system according to an aspect of the present invention includes: self-propelled transport device for transporting cells or materials to be used for culture operation; a plurality of treatment units for performing culture operation using the cells and materials; and transport command unit for commanding to move the self-propelled transport device, the cells and materials being transported to a required treatment unit through the self-propelled transport device, and is characterized in that the transport command unit includes a plurality of driving parameters having different acceleration and deceleration patterns, uses cell driving parameters when the cells are transported, and uses material driving parameters that move the self-propelled transport device at a speed higher than with the cell driving parameters, when the materials are transported.

According to this invention, as for cells, the self-propelled transport device is controlled using the cell driving parameters, which prevents stress on the cells being transported, and prevents the culture broth from spilling out of the culture vessel.

As for materials, the self-propelled transport device is controlled using the material driving parameters, which allows the materials to be transported quickly, reduces the time loss due to the supply of the materials, and enables efficient culture operation.

### BRIEF DESCRIPTION OF THE DRAWINGS

FIG. 1 is a configuration diagram of a cell culture system according to a first embodiment;
FIG. 2 is a control configuration diagram showing the relationship between a controller and each treatment unit or self-propelled transport device; and
FIG. 3 is a configuration diagram of a cell culture system according to a second embodiment.

### DETAILED DESCRIPTION OF THE PREFERRED EMBODIMENTS

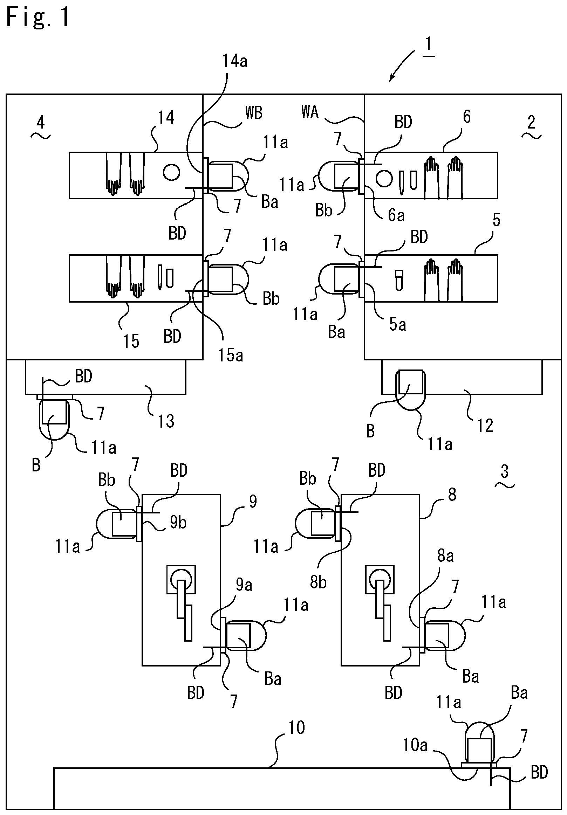

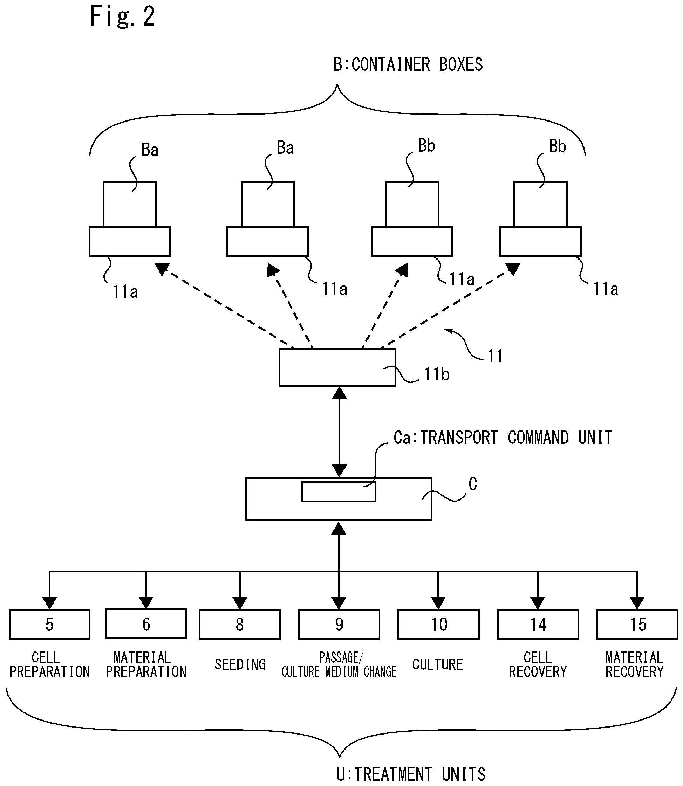

Explaining the illustrated embodiments below, FIG. 1 shows a cell culture system 1 for culturing cells in which cells are cultured by a series of culture operations consisting of a plurality of treatments on cells recovered from a patient. As shown in FIG. 2, the cell culture system 1 is designed to be entirely monitored and controlled by a controller C including a computer or PLC.

The aforementioned culture operations include a cell preparation treatment for making, as preparation, cells to be cultured suitable for treatment, material preparation treatment for preparing instruments and culture vessels required for culture, a culture broth, and the like, seeding treatment for seeding cells in a required culture vessel, culture treatment for culturing the cells in the culture vessel, passage/medium exchange treatment for passage and medium exchange for cells in culture, cell recovery treatment for recovering cells after culture, and material recovery treatment for recovering materials after use.

Note that the treatment in the aforementioned culture operation is merely illustrative, and other conceivable examples of the treatment include artificial proliferation (culture)/differentiation of cells, cell line generation, chemical treatment for cell activation, biological property modifying treatment, and treatment for combining with non-cell components, or subjecting genetic engineering modifications, and the cell culture system 1 according to the present invention is not limited to the treatments described in the following embodiments.

The facility constituting the cell culture system 1 includes a preparation operation room 2 for the cell preparation treatment and material preparation treatment, an aseptic operation room 3 in which the seeding treatment, culture treatment, passage/medium exchange treatment are automatically performed in an aseptic environment, and a recovery operation room 4 for the cell recovery treatment and material recovery treatment.

The insides of the preparation operation room 2, aseptic operation room 3, and recovery operation room 4 are decontaminated periodically and maintained at a higher pressure than the outside of the facility constituting the cell culture system 1 so that the clean room has a required air cleanliness. The aseptic operation room 3 is maintained at a higher pressure than the preparation operation room 2 and the recovery operation room 4 and set at a high air cleanliness.

The preparation operation room 2 is equipped with, as treatment units U, a cell supply unit 5 for performing the cell preparation treatment and a material supply unit 6 for performing the material preparation treatment.

The cell supply unit 5 and the material supply unit 6 can use known isolators and their inside can be decontaminated with decontamination device (not shown in the drawings) and are maintained at a positive pressure with aseptic air supply device (not shown in the drawings) to be maintained in a aseptic state.

Gloves wearable by an operator and a pass box (not shown in the drawings) are provided on the side surfaces of the cell supply unit 5 and the material supply unit 6.

To introduce cells or the aforementioned materials into the cell supply unit 5 and the material supply unit 6 from the outside, their outer surfaces are decontaminated inside the pass box, and they are then moved to the internal spaces of the cell supply unit 5 and the material supply unit 6.

One side surface of the cell supply unit 5 is in contact with a wall WA that separates the preparation operation room 2 and the aseptic operation room 3, and a connection port 5a is formed on the side surface of the cell supply unit 5. The connection port 5a can be opened and closed through a shutter not shown in the drawing.

A connector 7 consisting of a cylindrical member surrounding the connection port 5a is provided on the outer surface of the cell supply unit 5, and the connector 7 can be placed in the aseptic operation room 3 through a window opened in the wall WA. As will be described in detail later, the cell container box Ba can be connected to the cell supply unit 5 from the inside of the aseptic operation room 3 via the connector 7.

This enables cell exchange between the cell supply unit 5 and the cell container box Ba while maintaining the aseptic state.

Similarly, one side surface of the material supply unit 6 is also in contact with the wall WA, and a connection port 6a is formed on the side surface of the material supply unit 6. The connection port 6a can be opened and closed through a shutter not shown in the drawing.

A connector 7 consisting of a cylindrical member surrounding the connection port 6a is provided on the outer surface of the material supply unit 6, and the connector 7 can be placed in the aseptic operation room 3 through a window opened in the wall WA. As will be described in detail later, the material container box Bb can be connected to the material supply unit 6 from the inside of the aseptic operation room 3 via the connector 7.

This enables material exchange between the material supply unit 6 and the material container box Bb while maintaining the aseptic state.

The cell container box Ba and the material container box Bb are containers that can be sealed by the opening/closing door BD. In this first embodiment, the container boxes B used as the cell container box Ba and the material container box Bb have the same configuration.

Consisting of these container boxes B, a plurality of cell container boxes Ba for containing cells and a plurality of material container boxes Bb for containing materials are placed in the aseptic operation room 3, and these are automatically movable by the self-propelled transport device 11 controlled by the controller C as shown in the control configuration of FIG. 2.

As for such cell container boxes Ba and material container boxes Bb, their opening/closing doors BD are smaller than the inner diameter of the cylindrical members constituting the connectors 7, and the cylindrical member of each connector 7 is configured to be in contact with the periphery of the corresponding opening/closing door BD.

The self-propelled transport device 11 moves the cell container boxes Ba and the material container boxes Bb to bring the peripheral surfaces of the opening/closing doors BD of the cell container boxes Ba and the material container boxes Bb into contact with the cylindrical members so that they are connected.

The shutters of the connection ports 5a and 6a of the cell supply unit 5 and the material supply unit 6 are opened in this state and the opening/closing doors BD of the cell container boxes Ba and the material container boxes Bb as the container boxes B are opened, which establishes a communication between each treatment unit U and the internal space of the corresponding container box B.

The aseptic operation room 3 is provided with, as the treatment units U, a first treatment unit 8 for seeding treatment, a second treatment unit 9 for the passage/medium exchange treatment, and an incubator 10 for the culture treatment.

The first and second treatment units 8 and 9 are each composed of an isolator whose inside is maintained in a aseptic state and treatment devices such as a robot provided inside the isolator.

The inside of each isolator can be decontaminated with decontamination device (not shown in the drawings) and is maintained at a positive pressure with aseptic air supply device (not shown in the drawings) to be maintained in a aseptic state.

The first and second treatment units 8 and 9 have cell connection ports 8a and 9a for connection to the cell container boxes Ba and material connection ports 8b and 9b for connection to the material container boxes Bb, can be opened and closed through a shutter (not shown in the drawing), and are also provided with connectors 7 consisting of cylindrical members surrounding the connection ports 8a, 8b, 9a, and 9b like the connection ports 5a and 6a of the cell supply unit 5 and the material supply unit 6.

The cell connection ports 8a and 9a can be connected to cell container boxes Ba via the connectors 7 like the connection port 5a of the cell supply unit 5, and the material connection ports 8b and 9b can be connected to material container boxes Bb via the connectors 7 like the connection port 6a of the material supply unit 6.

The treatment devices provided inside the first and second treatment units 8 and 9 include, in addition to the robot, transport device for delivering cells and materials to the robot, dispensing device for dispensing a culture broth into a culture vessel, and centrifugation device for centrifuging a cell suspension containing cells, and may also include other appropriate devices depending on the required treatment.

In particular, the transport device is also configured to, for example, charge and discharge cells or materials between the cell container boxes Ba and the material container boxes Bb connected to the cell connection ports 8a and 9a and the material connection ports 8b and 9b.

The execution of these treatments is controlled by the controller C, and the operations required for the cell culture operation are automatically performed according to a preset program.

The incubator 10 houses a culture vessel containing cells, and adjusts the internal environment to a predetermined temperature and humidity through environment maintenance device (not shown in the drawing) to maintain the environment suitable for cell culture.

The incubator 10 is also provided with a cell connection port 10a that is the same as the cell connection ports 8a and 9a of the first and second treatment units 8 and 9. The cell connection port 10a can be opened and closed through a shutter (not shown in the drawing) and can be connected to the cell container box Ba via the connector 7.

Transport device (not shown in the drawing) is provided inside the incubator 10, moves the culture vessel to and from the cell container box Ba, moves the culture vessel to a required container position in the incubator 10, and transfers the culture vessel in which culture has ended is transferred to the cell container box Ba.

The recovery operation room 4 has, as treatment units U, a cell recovery unit 14 for cell recovery treatment and a material recovery unit 15 for the material recovery treatment.

Like the material supply unit 6 and the cell supply unit 5, the cell recovery unit 14 and the material recovery unit 15 are known isolators, and their insides can be decontaminated and are maintained in an aseptic state, and gloves wearable by an operator are provided on their side surfaces.

One side surface of the cell recovery unit 14 is in contact with a wall WB that partitions the recovery operation room 4 and the aseptic operation room 3, and a connection port 14a is formed on the side surface of the cell recovery unit 14. The connection port 14a can be opened and closed through a shutter not shown in the drawing.

The connection port 14a of the cell recovery unit 14 can also be connected to the cell container box Ba via the connector 7 like the connection port 5a of the cell supply unit 5, and enables cell exchange between the cell recovery unit 14 and the cell container box Ba while maintaining the aseptic state.

Similarly, one side surface of the material recovery unit 15 is in contact with the wall WB, and a connection port 15a is formed on the side surface of the material recovery unit 15. The connection port 15a can be opened and closed through a shutter not shown in the drawing.

The connection port 15a of the material recovery unit 15 can also be connected to the material container box Bb via the connector 7 like the connection port 6a of the material supply unit 6, and enables material exchange between the material recovery unit 15 and the material container box Bb while maintaining the aseptic state.

To explain the self-propelled transport device 11 now, the self-propelled transport device 11 comprises a plurality of transport vehicles 11a and a controller 11b that controls the movement of each transport vehicle 11a, and each cell container box Ba and material container box Bb move while being supported by a transport vehicle 11a that is self-propellable in the aseptic operation room 3.

As shown in FIG. 2, all the transport vehicles 11a are wirelessly controlled by a common controller 11b, and the controller 11b is instructed to move with the transport command unit Ca provided in the controller C.

The transport command unit Ca recognizes the positions of all the transport vehicles 11a located inside the aseptic operation room 3 and whether each transport vehicle 11a is used for moving the cell container box Ba for cells or whether it is used for moving the material container box Bb for materials.

The transport command unit Ca sequentially transmits a control command of each transport vehicle 11a to the controller 11b according to a preset program, and the controller 11b controls the movement of each transport vehicle 11a according to the command.

Upon reception of the control command from the transport command unit Ca, the controller 11b moves each transport vehicle 11a according to driving parameters consisting of preset acceleration, deceleration (negative acceleration), and transport speed.

In this embodiment, an autonomous intelligent vehicle (AIV) that can autonomously move is used as such self-propelled transport device 11.

The aseptic operation room 3 is provided with a charging station 12 for charging the transport vehicles 11a, and a decontamination station 13 for decontaminating the insides of the container boxes B as the cell container boxes Ba and the material container boxes Bb.

The charging station 12 is configured to wirelessly supply power to a plurality of transport vehicles 11a, and the transport command unit Ca can move, for charging, the transport vehicles 11a to the charging station 12 at an appropriate time while recognizing the charging status of each transport vehicle 11a.

The decontamination station 13 is connectable to the plurality of container boxes B via the connectors 7 like the treatment units U, and supplies a decontamination medium such as hydrogen peroxide vapor into each container box B to decontaminate the internal space.

Here, when cells and materials are transported using self-propelled transport device 11, vibrations due to the driving of the transport vehicle 11a and inertial force due to acceleration/deceleration act on the transported cells and materials.

Especially as for cells, since shock due to vibrations or acceleration/deceleration may stress the cells and a culture vessel containing the cells is not sealed, the culture broth may spill out of the culture vessel due to vibrations or acceleration/deceleration.

For this reason, when the culture vessel containing the cells is transported, in order to prevent stress due to the vibrations and acceleration/deceleration and spillage of the culture broth, acceleration and deceleration need to be kept low and the transport speed needs to be kept low for gentle movement.

On the other hand, as for materials used for the culture operation, as long as they are kept aligned so that they can be taken in and out by the transport device when connected to each treatment unit U, acceleration and deceleration can be increased without damaging the materials, and they can be controlled to move faster than the cell containers being transported.

Accordingly, in this embodiment, when the cells and materials are transported by the self-propelled transport device 11, the movement of the cells is controlled using the cell driving parameters, and the movement of the materials is controlled using the material driving parameters.

The driving parameters can be set as, for example, acceleration for accelerating to a predetermined speed of the driving of a transport vehicle 11a, deceleration for decelerating to a predetermined speed, and a transport speed, and changing these allows the vibration and inertial force during the driving of the transport vehicle 11a to be controlled.

To be specific, a transport vehicle 11a supporting a container box B (cell container box Ba) containing the culture vessel containing the cells is controlled based on the cell driving parameters, acceleration, deceleration, and transport speed are set low enough not to cause stress to the cells and spillage of the culture broth.

On the other hand, for a transport vehicle 11a supporting a container box B (material container box Bb) containing the material, control based on the material driving parameters is performed, and a high acceleration and deceleration that are made higher than the acceleration and deceleration in the cell driving parameters but to the extent that the material does not tip over due to vibrations or inertial force or the aligned state is not impaired, and a high transport speed are set to move the transport vehicle 11a more quickly than with the cell driving parameters.

Regarding the specific values of the cell driving parameters and the material driving parameters, the transport vehicle 11a may be moved while the cell container boxes Ba and the material container boxes Bb are actually containing culture vessels containing cells and materials, and the optimum acceleration, deceleration, and transport speed may be selected.

Here, when the cell container box Ba is empty, it is not necessary to run the transport vehicle 11a using the cell driving parameters, and the cell container box Ba can be moved more efficiently by control based on the material driving parameters.

In addition, in this embodiment, in case that not only the cell container box Ba but the material container box Bb gets empty, forwarding driving parameters in which the acceleration and deceleration are set higher than with the material driving parameters and the transport speed is set high are set in addition to the cell driving parameters and material driving parameters so that the transport vehicle 11a can be moved more quickly than with the material driving parameters, which further increases efficiency.

Further, a plurality of driving parameters consisting of different patterns of acceleration and deceleration based on these acceleration, deceleration (negative acceleration), and transport speed are saved in a storage area in the transport command unit Ca of the controller C and are read when needed.

A plurality of driving parameters consisting of different patterns of cell, material, and forwarding accelerations and decelerations are transmitted in advance from the transport command unit Ca to the controller 11b for each transport vehicle 11a.

Upon transmission of a control command from the transport command unit Ca, the controller 11b controls the movement of the corresponding transport vehicle 11a using the set driving parameters according to the commanded time information and positional information so that it moves to the commanded destination position at the commanded time.

The driving parameters to be used can be changed according to the control command from the transport command unit Ca, and the transport vehicle 11a is driven slowly, quickly, or more quickly according to the application.

Note that the control command consisting of the time information and the positional information transmitted from the transport command unit Ca to the controller 11b may include information on the driving parameters used each time. Also, driving parameters, which are defined as a combination of acceleration, deceleration, and transport speed in this embodiment, may consist of acceleration and deceleration, and the transport speed may be commanded each time in combination with time information and positional information.

The procedure of the aforementioned culture operation using the cell culture system 1 with the aforementioned configuration will now be explained. FIG. 1 shows a state during the culture operation and shows a state in which a container box B is connected to each treatment unit U, but at the start of the operation, each cell container box Ba and material container box Bb are separated from the corresponding treatment unit U.

The internal spaces of all the container boxes B are decontaminated in advance at the decontamination station 13, and the charging of each transport vehicle 11a is completed at the charging station 12.

In the controller C, an operation control program for each treatment unit U for the current culture operation and a movement control program for the related transport vehicle 11a are preset.

Upon start of the culture operation in the cell culture system 1, the transport command unit Ca moves the transport vehicle 11a specified by the program to the position of the connection port 5a of the cell supply unit 5 in the wall WA between the preparation operation room 2 and the aseptic operation room 3, according to the movement control program set in the controller C. Consequently, the cell container box Ba supported by the corresponding transport vehicle 11a is connected to the connection port 5a via the connector 7.

Similarly, the transport command unit Ca moves the transport vehicle 11a specified by the program to the position of the connection port 6a of the material supply unit 6 in the wall WA, according to the movement control program. Consequently, the material container box Bb supported by the corresponding transport vehicle 11a is connected to the connection port 6a via the connector 7.

In the movement of each transport vehicle 11a at this time, since the cell container box Ba and the material container box Bb are both empty (do not contain anything), the transport command unit Ca moves each transport vehicle 11a at the highest speed and quickly by using the forwarding driving parameters.

On the other hand, when cells to be cultured are carried into the preparation operation room 2 from the outside, the operator moves the cells into the cell supply unit 5 via a pass box (not shown in the drawing).

Subsequently, the operator uses the cell supply unit 5 to perform an operation that allows the cells contained in the required container to be introduced in a centrifuge tube in the form of a suspension that can be processed in the first treatment unit 8.

Upon completion of this operation, the operator transfers the centrifuge tube containing the cells into the cell container box Ba connected to the cell supply unit 5 via the connection port 5a, and then closes the shutter of the connection port 5a and the opening/closing door BD of the cell container box Ba.

Upon reception of the notification that the shutter or the opening/closing door BD has been closed or the operation has been completed by the operator, the controller C moves the transport vehicle 11a supporting the cell container box Ba to the cell connection port 8a of the first treatment unit 8.

At this time, the transport command unit Ca changes the forwarding driving parameters to the cell driving parameters, and moves the transport vehicle 11a supporting the cell container box Ba containing the container (centrifuge tube) containing the cells according to the cell driving parameters. Consequently, the cells in the cell container box Ba are not stressed by the gentle movement of the transport vehicle 11a.

On the other hand, materials used for the culture operation, for example, in the form of packages each including multiple culture vessels or pipettes, are carried into the preparation operation room 2 from the outside.

The operator moves the materials into the material supply unit 6 via a pass box (not shown in the drawing), opens the packages inside the material supply unit 6 to take out the materials, and assorts the culture vessels and pipettes into numbers required for the treatment to be performed in the first treatment unit 8 and the second treatment unit 9.

After that, the operator transfers the materials to be used in the first treatment unit 8 to the material container box Bb connected to the material supply unit 6 via the connection port 6a, and at that time, each material is transferred to a predetermined position in the material container box Bb so that the transport device can hold each material in the first treatment unit 8.

Upon completion of material transfer to the material container box Bb, the controller C moves the transport vehicle 11a supporting the material container box Bb to the position of the material connection port 8b of the first treatment unit 8.

At this time, the transport command unit Ca changes the forwarding driving parameters to the material driving parameters, and moves the transport vehicle 11a supporting the material container box Bb containing the materials according to the material driving parameters. Hence, the materials do not tip over inside the material container box Bb, allowing it to move faster than the transport vehicle 11a supporting the cell container box Ba containing the cells in such a manner that the assorted materials do not move.

Subsequently, when the cell container box Ba is connected to the cell connection port 8a of the first treatment unit 8 via the connector 7, the shutter (not shown in the drawing) and the opening/closing door BD are opened and the centrifuge tube containing cells in the cell container box Ba is taken into the first treatment unit 8.

Similarly, when the material container box Bb is connected to the material connection port 8b of the first treatment unit 8 via the connector 7, the shutter (not shown in the drawing) and the opening/closing door BD are opened and the materials in the material container box Bb are taken into the first treatment unit 8.

When the centrifuge tube, the culture vessels, and the like is taken into the first treatment unit 8 in this way, in the first treatment unit 8, the robot and the transport device controlled by the controller C use the supplied cells and materials to automatically seed the cells in the culture vessels.

Note that the material transport operation by the material container box Bb is performed by a transport vehicle 11a supporting another material container box Bb in the second treatment unit 9 following the first treatment unit 8.

Upon completion of this seeding treatment, the culture vessels in which the cells are seeded are transferred from the first treatment unit 8 to the cell container box Ba by the transport device, and the used materials are transferred from the first treatment unit 8 to the material container box Bb.

When the opening/closing doors BD of the cell container box Ba and the material container box Bb are closed, the controller C recognizes the completion of the seeding operation and moves the transport vehicle 11a supporting the cell container box Ba containing the culture vessels in which the cells have been seeded, from the first treatment unit 8 to the position of the connection port 10a of the incubator 10.

At this time, the transport command unit Ca moves the transport vehicle 11a according to the cell driving parameters, so that the culture vessels containing cells contained in the cell container box Ba are gently transported, reducing the stress on the cells and preventing the spillage of the culture broth from the culture vessels.

The cell container box Ba detached from the first treatment unit 8 then moves to the incubator 10 and is connected to the connection port 10a via the connector 7. After that, the shutter and the opening/closing door BD are opened, and the culture vessels containing cells in the cell container box Ba are taken into the incubator 10.

Cell culture is started in the culture vessels in the incubator 10, but a predetermined time is required for cell culture in the incubator 10. Hence, if the culture vessels are transferred to the incubator 10, the empty cell container box Ba is detached from the incubator 10.

In other words, when the opening/closing door BD of the cell container box Ba is closed, the controller C moves the transport vehicle 11a from the incubator 10 through the transport command unit Ca, and the transport command unit Ca changes the cell driving parameters to the forwarding driving parameters and moves the transport vehicle 11a supporting the empty cell container box Ba according to the forwarding driving parameters.

The transport vehicle 11a is moved at a speed higher than with the material driving parameters, the cell container box Ba is moved to the decontamination station 13 to undergo internal decontamination, and the transport vehicle 11a then moves to the charging station 12 for charging as needed and goes into a standby mode.

On the other hand, when the used materials that were used in the first treatment unit 8 are stored in the material container box Bb and the opening/closing door BD is closed, the transport command unit Ca moves the transport vehicle 11a supporting the material container box Bb to the position of the connection port 15a of the material recovery unit 15 of the recovery operation room 4. After that, the material container box Bb is connected to the connection port 15a of the material recovery unit 15 via the connector 7.

At this time, the controller 11b controls the movement of the transport vehicle 11a according to the material driving parameters that are still selected, whereby the transport vehicle 11a can move at a speed higher than with the cell driving parameters.

In the material recovery unit 15, when the operator opens the opening/closing door BD of the material container box Bb, the used materials in the material container box Bb are transferred into the material recovery unit 15 and are discarded according to a required procedure.

When the material container box Bb gets empty, the transport command unit Ca moves the transport vehicle 11a supporting the empty material container box Bb to the decontamination station 13, but at this time, changes the driving parameters to the forwarding driving parameters in order to move it at a speed higher than with the material driving parameters. At the decontamination station 13, the inside of the empty material container box Bb is decontaminated.

Upon completion of the decontamination, as necessary, the transport command unit Ca moves the transport vehicle 11a to the charging station 12 to charge the vehicle, and the transport vehicle 11a goes into the standby mode.

After the cells are cultured in the incubator 10 for a predetermined length of time, the cells are subjected to passage treatment for dividing into a plurality of culture vessels and a culture medium exchange treatment for exchanging the culture broth in the culture vessels.

To explain the passage treatment, after a predetermined time has elapsed since the culture vessels containing cells were stored in the incubator 10, the controller C moves the designated transport vehicle 11a supporting the empty container box B to the incubator 10. At this time, the transport command unit Ca transmits a control command to the controller Cb to control the movement according to the forwarding driving parameters.

The empty container box B as a cell container box Ba is connected to the connection port 10a of the incubator 10, and the culture vessels containing the cultured cells are housed in the cell container box Ba and the opening/closing door BD is closed. Then, the transport command unit Ca changes the forwarding driving parameters to the cell driving parameters and moves the transport vehicle 11a supporting the cell container box Ba to the position of the cell connection port 9a of the second treatment unit 9.

When the cell container box Ba is connected to the cell connection port 9a of the second treatment unit 9 in this way, the controller C controls the operation of the transport device, the robot, and the like of the second treatment unit 9 and first counts the number of cells in the culture vessels.

The controller C automatically determines the number of culture vessels to undergo passage according to the counted number of cells and performs cell passage operation on that number of culture vessels by controlling the robot and the like.

Upon completion of this passage treatment, the culture vessels in which the cells have been seeded by the passage are transferred from the second treatment unit 9 to the cell container box Ba, and the used materials are transferred from the second treatment unit 9 to the material container box Bb.

Similarly to the seeding operation in the first treatment unit 8, upon completion of the passage operation, the controller C moves the transport vehicle 11a supporting the cell container box Ba containing the culture vessels in which the cells have been seeded, to the position of the connection port 10a of the incubator 10 through movement control according to the cell driving parameters, and, similarly to the aforementioned procedure, connects the cell container box Ba to it via the connector 7 to transfer the culture vessels to the incubator 10 for performing culture for a predetermined time.

Upon completion of the passage treatment in the second treatment unit 9, like the used materials in the first treatment unit 8, the used materials that have been used for the passage treatment are transferred from the second treatment unit 9 to the material container box Bb and then transported to the material recovery unit 15 by the movement of the transport vehicle 11a based on the material driving parameters to undergo recovery treatment.

Finally, all the culture vessels that have undergone culture for a predetermined length of time in the incubator 10 are transported to the cell recovery unit 14 for recovery treatment. At this time, like in the previous transportation from the incubator 10 to the second treatment unit 9, the culture vessel is transported by moving the transport vehicle 11a according to the cell driving parameters.

When the cell container box Ba containing the culture vessels after culture is moved to the cell recovery unit 14, the cell container box Ba is connected to the connection port 14a, and the operator takes out the culture vessels from the cell container box Ba to subject them to recovery treatment, and then takes them out of the recovery operation room 4.

In the case of the cell culture system 1 with this configuration, for example, in the passage treatment in the second treatment unit 9, the number of culture vessels to undergo passage varies depending on the number of cultured cells, so that the time required for the treatment varies.

The transport vehicle 11a for transporting each container box B can be prepared independently for each transport route between the treatment units U, although this is uneconomical because many transport vehicles 11a are needed. For this reason, in this embodiment, a program is set in the controller C to manage the transport vehicles 11a in such a manner that the transport vehicles 11a that have been transported as the minimum number of transport vehicles 11a are used in another transport route.

In particular, since the transport vehicle 11a can be used for both cell transport and material transport by changing the driving parameters, the required number of transport vehicles 11a can be reduced by using them properly according to the situation.

When the time required for treatment fluctuates as in the passage operation in the second treatment unit 9, the subsequent schedule is changed and a program is reset in the controller C.

Further, automating the transportation using the self-propelled transport device 11 can make the aseptic operation room 3 unmanned, thereby reducing the number of operators engaged in the operation and also maintaining the aseptic state and cleanliness in the environment.

Since AIV is adopted as the self-propelled transport device, it is not necessary to set any movement route for the transport vehicles 11a in the aseptic operation room 3 or install guides or sensors in the aseptic operation room 3, and it is easy to install the configuration according to the present invention in an existing facility.

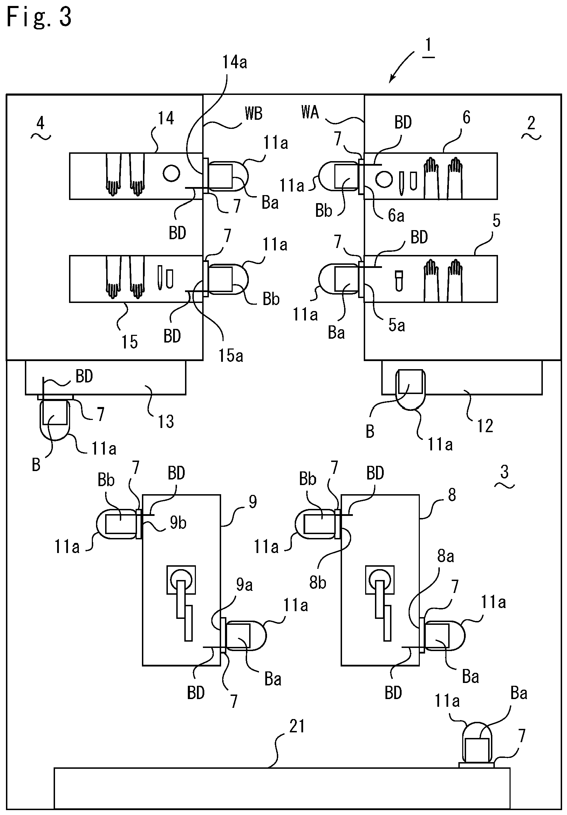

FIG. 3 shows a configuration diagram of a cell culture system 1 according to the second embodiment. A part of its configuration different from that of the first embodiment will be described below, and description of the other part common to the first embodiment will be omitted.

In the second embodiment, as container boxes B, the cell container boxes Ba containing the cells and the material container boxes Bb containing materials have different configurations. To be specific, the cell container box Ba itself has a function as an incubator.

In the aseptic operation room 3 of this embodiment, a culture station 21 is provided instead of the incubator 10 of the first embodiment.

This culture station 21 is connected to the cell container box Ba configured to be an incubator to be supplied with electric power, gas, and the like required for culture.

In particular, in the culture station 21 of this embodiment, the cell container box Ba stands for a predetermined length of time with the opening/closing door BD of the cell container box Ba left closed, so that the cells are cultured inside the cell container box Ba.

In the second embodiment configured in this way, the cell container box Ba is moved and connected to the culture station 21 to perform culture, but the container box B is dedicated for cells and the transport vehicle 11a that supports it is also dedicated for cells and they cannot be moved from the culture station 21 during culture.

Hence, more transport vehicles 11a are required than in the case of the first embodiment, but the driving parameters are not switched for cells and materials, facilitating the program and control.

It is also possible to detach the cell container box Ba from the transport vehicle 11a at the culture station 21 and use the transport vehicle 11a for transport for another treatment. In this case, the decontamination station 13 is used to store an empty cell container box Ba and material container box Bb and they are supported by the transport vehicle 11a that does not support anything.

A cell culture system 1 according to the third embodiment will now be described. A part of its configuration different from that of the first embodiment will be described below, and description of the other part common to the first embodiment will be omitted.

In the third embodiment, like in the first embodiment, as container boxes B, the cell container boxes Ba containing the cells and the material container boxes Bb containing materials have the same configuration. To be specific, a container box B configured to allow outside air to flow in through a cleaning filter such as a HEPA filter is used.

With use of this container box B, if it stores a culture vessel containing cells as a cell container box Ba, the cells can be cultured while the cell container box Ba itself is stored in the incubator 10, and when the transport vehicle 11a supports the cell container box Ba and moves it to the incubator 10, the culture vessel containing the cells are supposed to be taken into the incubator 10 while being in the cell container box Ba through transport device provided to the incubator 10.

In this embodiment, after the cell container box Ba is taken into the incubator 10, the transport vehicle 11a that has transported the cell container box Ba supports another cell container box Ba or material container box Bb stored in the decontamination station 13 and performs transport operation on the other treatment unit U.

Although the container boxes B are shared for cells and materials in the third embodiment, it is also possible to provide a cleaning filter to only those for cells so that they can be dedicated as in the second embodiment.

A cell culture system 1 according to the fourth embodiment will now be described. A part of its configuration different from that of the first to third embodiments will be described below, and description of the other part common to the first to third embodiments will be omitted.

This fourth embodiment assumes transportation for simultaneously supplying cells and materials to a treatment unit U, and uses a common container box B capable of containing cells and materials, so that the cells and materials are contained in one container box B and transported at the same time.

In particular, the transport vehicle 11a is moved to the cell supply unit 5 and then to the material supply unit 6 to connect the container box B to them so that the cells (centrifuge tube containing the cell suspension) and various materials can be contained in the container box B.

After that, the transport vehicle 11a is moved to the cell connection port 8a and then to the material connection port 8b of the first treatment unit 8 to connect the container box B to them so that the contained cells and materials can be carried into the first treatment unit 8.

At this time, the transport vehicle 11a is moved under control based on the material parameters when only materials are contained in the container box B, and is moved under control based on the cell parameters when cells and materials or only cells are contained.

When a common container box B for cells and materials is used as in the fourth embodiment, the cell supply unit 5 and the material supply unit 6 are used as common treatment units U, and in the first treatment unit 8 and the second treatment unit 9, the cell connection ports 8a and 9a and the material connection ports 8b and 9b can be used as common connection ports so that cells and materials can be taken in and out through the common connection ports for each treatment unit U.

In the above embodiments, the aseptic operation room 3 is provided with the first and second treatment units 8 and 9 as treatment units U to perform the culture operation as described above; however, more treatment units U may be provided to further subdivide the culture operation, and for example, a treatment unit U for performing operation such as gene manipulation may be provided.

To be specific, for example, a plurality of second treatment units 9 may be provided, and the passage treatment and the culture medium exchange treatment may be performed concurrently using a plurality of second treatment units 9, and a third and fourth treatment units U configured to perform other treatments may be provided.

### Reference Signs List

- 1: Cell culture system
- 2: Preparation operation room
- 3: Aseptic operation room
- 4: Recovery operation room
- 5: Cell supply unit
- 6: Material supply unit
- 7: Connector
- 8: First treatment unit
- 9: Second treatment unit
- 8a, 9a: Cell connection ports
- 8b, 9b: Material connection ports
- 10: Incubator
- 11: Self-propelled transport device
- 11a: Transport vehicle
- 11b: Controller
- 14: Cell recovery unit
- 15: Material recovery unit
- U: Treatment unit
- B: Container box
- Ba: Cell container box
- Bb: Material container box
- C: Controller
- Ca: Transport command unit

## Claims

1. A cell culture system comprising:
self-propelled transport device for transporting cells or materials to be used for culture operation;
a plurality of treatment units for performing culture operation using the cells and materials; and
transport command unit for commanding to move the self-propelled transport device,
the cells and materials being transported to a required treatment unit through the self-propelled transport device, the cell culture system being **characterized in that**
the transport command unit includes a plurality of driving parameters having different acceleration and deceleration patterns, uses cell driving parameters when the cells are transported, and uses material driving parameters that move the self-propelled transport device at a speed higher than with the cell driving parameters, when the materials are transported.

2. The cell culture system according to Claim 1, **characterized in that** the transport command unit includes, as the driving parameters, forwarding driving parameters that move the self-propelled transport device at a speed higher than the material driving parameters, and uses the forwarding driving parameters when the self-propelled transport device is moved without transporting the cells or materials.

3. The cell culture system according to Claim 1 or 2, **characterized by** further comprising a container box that contains the cells or materials, wherein the self-propelled transport device includes a plurality of self-propelled transport vehicles and a controller for controlling the movement of each transport vehicle, and the container box is transported while being supported by the transport vehicle.
